# EUROPEAN PATENT APPLICATION

(11) **EP 4 449 990 A1**
(43) Date of publication of application: **23.10.2024**
(21) Application number: 22907144.4
(22) Date of filing: 21.11.2022
(51) Int. Cl.: A61B 5/256, A61B 5/291

(54) **BRAIN WAVE MEASURING DEVICE AND BRAIN WAVE MEASURING METHOD**

(30) Priority: 15.12.2021 JP 2021203139
(71) Applicant: Sumitomo Bakelite Co.Ltd., Shinagawa-ku Tokyo 140-0002 (JP)
(72) Inventor: KITAZOE, Katsuma, Tokyo 140-0002 (JP); ISHIKAWA, Eiji, Tokyo 140-0002 (JP)
(74) Representative: Ter Meer Steinmeister & Partner
(86) International application number: PCT/JP2022/043055
(87) International publication number: WO 2023/112613

(57) **Abstract**

A brain wave measuring device (1) includes a frame (20) that is mounted on a head (99) of a subject, an electrode part (10) that comes into contact with the head (99), and an elastic electrode fixing member (70) that is attached to the frame (20) and to which the electrode part (10) is attached, in which the electrode fixing member (70) expands along a shape of the head (99) in a case where the frame (20) is mounted on the head (99) and the electrode part (10) is pushed against the head (99).

## Description

### TECHNICAL FIELD

The present invention relates to a brain wave measuring device and a brain wave measuring method.

### BACKGROUND ART

Until now, various developments have been made regarding brain wave measuring devices. As this kind of technology, for example, a technology disclosed in Patent Document 1 is known. The electrode for brain wave measurement (brain wave electrode holding tool) disclosed in Patent Document 1 is a brain wave electrode holding tool, including a main portion that is arranged around a head, a plurality of support portions that are attached to the main portion, and a brain wave electrode that is provided on at least a part of the support portions on a distal end side and is supported inside the main portion, in which the main portion has an opening portion having a size capable of allowing a finger to be inserted and to touch the brain wave electrode, the brain wave electrode has a flexible portion of which a proximal end side is supported by the support portion and that is elastically deformed with flexibility, and an electrode part that is provided on a distal end side of the flexible portion and that is brought into contact with a head surface, the flexible portion is deformable until the electrode part is moved in a direction away from an axis of the support portion, the flexible portion applies a contact pressure between the electrode part and the head surface, and the flexible portion applies the contact pressure between the electrode part moved in a substantially parallel direction along the head surface and the head surface.

### RELATED DOCUMENT

### PATENT DOCUMENT

[Patent Document 1] Japanese Unexamined Patent Publication No. 2018-94054

### SUMMARY OF THE INVENTION

### TECHNICAL PROBLEM

In the technique disclosed in Patent Document 1, it is necessary to adjust the contact state with the scalp for each of the plurality of electrode parts, and the adjustment takes time. Therefore, another technique for shortening the adjustment time has been demanded.

The present invention has been made in view of such circumstances, and an object of the present invention is to provide a technique for facilitating an operation of adjusting a mounting state in a brain wave measuring device.

### SOLUTION TO PROBLEM

According to the present invention, the following techniques are provided.
[1] A brain wave measuring device including a frame that is mounted on a head of a subject, an electrode part that comes into contact with the head, and an elastic electrode fixing member that is attached to the frame and to which the electrode part is attached, in which the elastic electrode fixing member expands along a shape of the head in a case where the frame is mounted on the head and the electrode part is pushed against the head.
[2] The brain wave measuring device according to [1], in which the elastic electrode fixing member has an elastic sheet material.
[3] The brain wave measuring device according to [1] or [2], in which the elastic electrode fixing member has an elastic band member.
[4] The brain wave measuring device according to [2] or [3], further including a fixing adjusting part that adjusts an expansion and contraction state of the elastic electrode fixing member and attaches the electrode fixing member to the frame.
[5] The brain wave measuring device according to [4], in which the fixing adjusting part has an insertion part that is provided in the frame and into which a part of the elastic electrode fixing member is inserted, and a fixing part that fixes the elastic electrode fixing member inserted into the insertion part to the frame.
[6] The brain wave measuring device according to any one of [1] to [5], in which the elastic electrode fixing member is attachable to and detachable from the frame, and an attachment position is adjustable.
[7] The brain wave measuring device according to any one of [1] to [6], in which the elastic electrode fixing member and the frame are integrally provided.
[8] The brain wave measuring device according to any one of [1] to [7], in which a portion that expands and contracts in the elastic electrode fixing member is provided with a silicone rubber.
[9] The brain wave measuring device according to any one of [1] to [8], further including a mounting adjusting part that adjusts a mounting state of the frame in a head circumference direction.
[10] A brain wave measuring method including performing a brain wave measurement by mounting the brain wave measuring device according to any one of [1] to [9] on a head of a subject.

### ADVANTAGEOUS EFFECTS OF INVENTION

According to the present invention, a technique of facilitating an operation of adjusting the mounting state in the brain wave measuring device can be provided.

### BRIEF DESCRIPTION OF THE DRAWINGS

Fig. 1 is a diagram schematically showing a brain wave measuring device according to a first embodiment in a state of being mounted on a head of a subject.
Fig. 2 is a diagram schematically showing the brain wave measuring device according to the first embodiment.
Fig. 3 is a diagram schematically showing a frame according to the first embodiment.
Fig. 4 is a perspective view of an electrode part according to the first embodiment.
Fig. 5 is a cross-sectional view schematically showing a state in which an electrode part is attached to an electrode fixing member in the brain wave measuring device according to the first embodiment.
Fig. 6 is a diagram schematically showing a brain wave measuring device including an electrode fixing member according to a modification example of the first embodiment.
Fig. 7 is a diagram schematically showing the front peripheral band member according to a modification example of the first embodiment.
Fig. 8 is a diagram schematically showing a brain wave measuring device according to a second embodiment in a state of being mounted on a head of a subject.
Fig. 9 is a plan view schematically showing an electrode fixing member according to the second embodiment.
Fig. 10 is a diagram schematically showing a fixing part according to the second embodiment.
Fig. 11 is a diagram schematically showing a fixed state of the fixing adjusting part according to the second embodiment.

### DESCRIPTION OF EMBODIMENTS

### <<First Embodiment>>

Hereinafter, embodiments of the present invention will be described with reference to drawings.

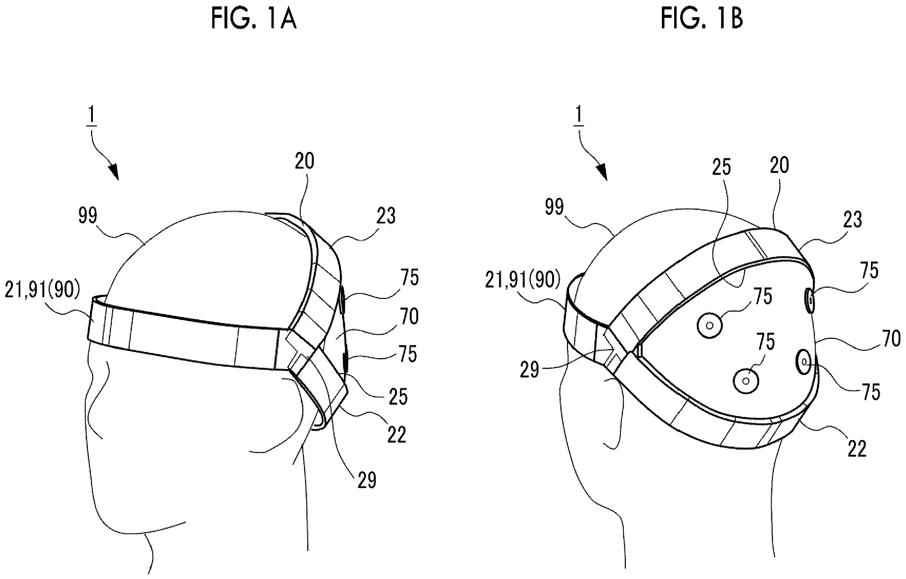

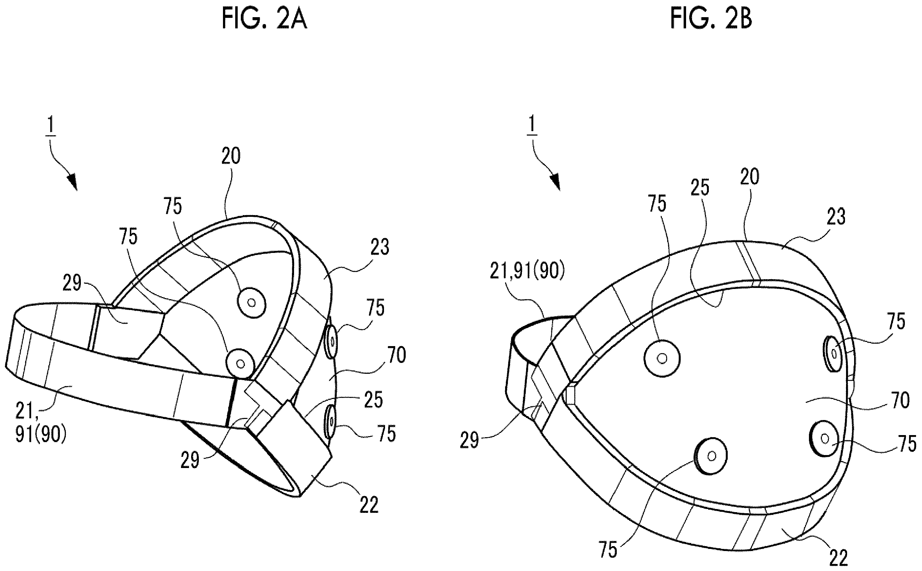

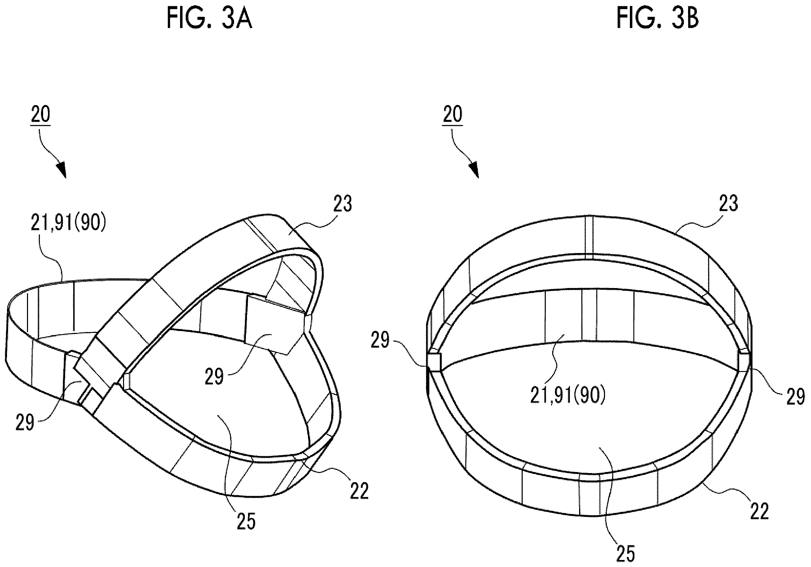

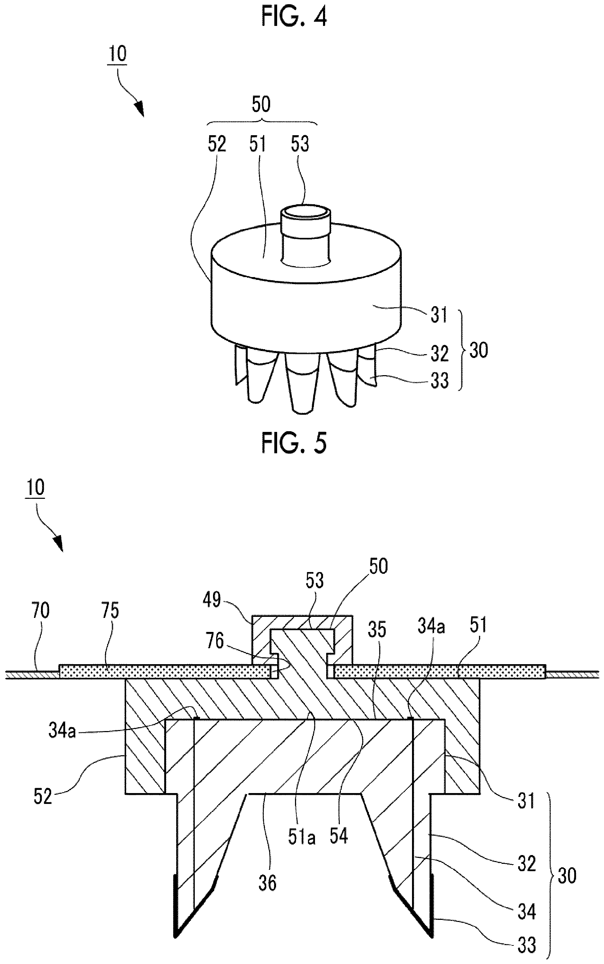

Fig. 1A and 1B are diagrams schematically showing the brain wave measuring device 1 in a state of being mounted on a head 99 of a subject, Fig. 1A is a perspective view as viewed from a front side, and Fig. 1B is a perspective view as viewed from a rear side. The electrode part 10 is removed in these diagrams. Figs. 2A and 2B are diagrams schematically showing the brain wave measuring device 1, Fig. 2A is a perspective view as viewed from a front side, and Fig. 2B is a perspective view as viewed from a rear side. Figs. 3A and 3B are diagrams schematically showing the frame 20, Fig. 3A is a perspective view as viewed from a rear side, and Fig. 3B is a perspective view as viewed from a rear side. Fig. 4 is a perspective view of the electrode part 10. Fig. 5 is a cross-sectional view schematically showing a state in which the electrode part 10 is attached to the electrode fixing member 70 in the brain wave measuring device 1. In the present embodiment, the inner side is described as a side facing (a side which comes into contact with) the head 99 in the frame 20, and the outer side is described as a side opposite to the inner side (a side not facing the head 99).

The brain wave measuring device 1 is mounted on a head 99 of a person, detects brain wave as potential fluctuation from a living body, and outputs the detected brain wave to a brain wave display device (not shown). The brain wave display device acquires the brain wave detected by the brain wave measuring device 1, and performs monitor display, data storage, and known brain wave analysis processing.

### <Structure of Brain Wave Measuring Device 1>

As shown in Figs. 1A and 1B, the brain wave measuring device 1 has a frame 20, a sheet-shaped electrode fixing member 70 attached to an opening 25 of the frame 20 on an occipital region side, and an electrode part 10 attached to the electrode fixing member 70. In a case where the frame 20 is mounted on the head 99 and the electrode part 10 is pushed against the head 99, the elastic electrode fixing member 70 expands along the shape of the head 99 (occipital region). As a result, the electrode part 10 attached to the electrode fixing member 70 is pushed against the head 99 with an appropriate force to the head 99 by the elastic force of the electrode fixing member 70. In addition, the frame 20 has a mounting adjusting part 90 that is provided in the frame 20 and that has an elastic member, and that is capable of adjusting a contact state of the electrode part 10 with the head 99. In the present embodiment, a configuration will be described in which a vertex mounting adjusting part 92 that mainly adjusts the mounting state in the head circumference direction is provided as the mounting adjusting part 90.

The description will be given below in more detail.

### <Structure of Frame 20>

The frame 20 is provided along the forehead, the temporal region, and the occipital region of the head 99 with a frame-shaped (band-shaped) hard member, a flexible member, an elastic member, or a combination thereof.

As the hard member, for example, a thermoplastic resin (ABS, PE, PA, PBT, PC, PEEK, PEI, PET, PMMA, POM, PP, PPS, or PS), a thermosetting resin (phenol resin, epoxy resin, urea resin, or melamine resin), a metal (aluminum, copper, or stainless steel), or the like can be used. As the flexible member, a PET film, a PE film, a PP film, a PVC film, a PEN film, a PI film, a plant fiber cloth (cotton or morning), an animal fiber cloth (silk, wool, alpaca, angora wool, cashmere, or mohair), a synthetic fiber cloth (PE, PA, PC, or PES), a carbon fiber cloth, an aramid fiber cloth, an aluminum plate, a SUS plate, a copper plate, or the like can be used. As the elastic member, silicone rubber, TPS, TPO, TPVC, TPU, TPEE, TPAE, natural rubber, synthetic rubber, chloroprene rubber, urethane rubber, or fluororubber can be used.

Specifically, the frame 20 has a front peripheral band member 21, a rear peripheral frame 22, and a top frame 23.

The front peripheral band member 21 and the rear peripheral frame 22 are connected to each other by two connecting parts 29 located on the temporal region of the head 99 in a state of being mounted, and are configured in an annular shape surrounding the periphery (forehead, temporal region, and occipital region) of the head 99 in a connected state. The top frame 23 is provided to extend out from the two connecting parts 29 described above and to be curved along the top end of the head 99.

The front peripheral band member 21 is provided to extend along the forehead of the frontal region, in other words, in the head circumference direction (forehead side), by a band-shaped member having elasticity.

The front peripheral band member 21 functions as a head circumference mounting adjusting part 91 that adjusts the mounting state in the head circumference direction to an appropriate tightness by expanding the front peripheral band member 21 according to the head circumference in a case in which the brain wave measuring device 1 is mounted to the head 99.

The material of the front peripheral band member 21 is not particularly limited as long as it can follow the shape of the head circumference and has elasticity, and for example, a rubber sheet such as a silicone sheet, or a rubber string (woven rubber, knitted rubber, or flat rubber such as elastic cord) may be used. In addition, in the front peripheral band member 21, a portion that expands and contracts may be a part thereof as long as it functions as the head circumference mounting adjusting part 91.

The rear peripheral frame 22 is provided in a frame shape (band shape) having a predetermined width to be curved along the shape of the occipital region with a hard member such as a polyamide resin.

The top frame 23 is provided in a frame shape (band shape) having a predetermined width to be curved along a shape from the temporal region to the parietal region with a hard member, for example, such as a polyamide resin in the same manner as the rear peripheral frame 22.

### <Electrode Fixing Member 70>

The opening 25 on the occipital region side is formed to be surrounded by the rear peripheral frame 22 and the top frame 23 described above. The electrode fixing member 70 is attached to the top frame 23 and the rear peripheral frame 22 to close the opening 25.

The electrode fixing member 70 is constituted to have an elastic sheet material. For example, the thickness of the sheet material is not particularly limited, but is preferably a thickness that is sufficiently expandable in a case of being mounted on the head 99 and that has a strength that is not damaged even in continuous use, and may be, for example, 0.1 to 2 mm.

As a material of the sheet material, for example, a rubber-like elastic member can be used. Specifically, the same material as the material of the electrode part 10 described below may be used.

The electrode fixing member 70 may be attachable to and detachable from the frame 20 (more specifically, the rear peripheral frame 22 or the top frame 23), and an attachment position may be adjustable. As the attachable and detachable mechanism, various mechanisms such as a hook and loop fastener mechanism, a button fitting mechanism, and a screw fitting mechanism may be adopted.

The electrode fixing member 70 and the frame 20 (rear peripheral frame 22 and top frame 23) may be integrally provided with the same material (for example, silicone rubber). In this case, for example, the portion serving as the frame 20 (rear peripheral frame 22 and top frame 23) has a structure that is a thick shape and does not substantially expand and contract, and the portion serving as the electrode fixing member 70 has a structure that is thin and expands and contracts.

### <Electrode Attachment Member 75>

The electrode fixing member 70 has a plurality of electrode attachment members 75 for attaching the electrode part 10 at a predetermined position (electrode position based on the 10-20 system). Here, as an example, the electrode attachment members 75 are provided at four positions of the electrode positions P3, P4, O1, and O2 according to the 10-20 system. The electrode attachment member 75 has a substantially doughnut shape with a through-hole 76 at the center in a top view. A male type snap button 53 of the electrode part 10, which will be described later, is inserted into the through-hole 76 from the inside to the outside.

### <Structure of Electrode Part 10>

Fig. 4 shows a perspective view of the electrode part 10. Fig. 5 is a cross-sectional view of the electrode part 10 in a state of being mounted on the electrode attachment member 75 of the electrode fixing member 70.

The electrode part 10 has an electrode part main body 30 and a cap-integrated type snap button 50. The cap-integrated type snap button 50 has conductivity, functions as a holder that has a bottomed cylindrical shape and accommodates the electrode part main body 30 therein, and functions as a connection part that is connected to the external measurement unit. The electrode part main body 30 is fixed inside a tubular shape of the cap-integrated type snap button 50 by a silicone adhesive or the like.

### <Electrode Part Main Body 30>

The electrode part main body 30 has a base 31, a projecting part 32, a conductive contact part 33, and a signal line part 34.

The base 31 and the projecting part 32 are integrally provided with a rubber-like elastic member. The specific material of the elastic member will be described later. The base 31 and the projecting part 32 are not limited to the configuration to be integrally provided, and may be configured by assembling separately provided base 31 and projecting part 32 by an adhesive or a fitting structure.

The base 31 has a substantially columnar shape. A plurality of the projecting parts 32 that have a substantially conical shape and protrude in a lower side direction in the drawing are provided on a circular base lower surface 36 of one end side (lower side in the drawing) of the base 31 at predetermined intervals in a circumferential direction. The shape of the projecting part 32 is not limited to the conical shape, and various shapes of a pyramid such as a triangular pyramid, a columnar shape, and the like may be adopted.

A conductive contact part 33 is provided on at least a distal end side surface of the projecting part 32. The conductive contact part 33 may be provided on the entire surface of the projecting part 32.

The outer diameter of the base 31 is, for example, 10 mm to 50 mm. The height (thickness) of the base 31 is, for example, 2 mm to 30 mm. The height of the projecting part 32 is, for example, 3 mm to 15 mm. The width (outer diameter of the root portion) of the projecting part 32 is, for example, 1 mm to 10 mm.

### <Structure of Signal Line Part 34>

As shown in Fig. 5, a signal line part 34 is provided in the electrode part main body 30 as a signal path connected to the conductive contact part 33. Various wiring structures can be adopted for the signal line part 34 as long as the signal line part 34 is in a state of being conducted through the base 31 and the projecting part 32. Here, the signal line part 34 is provided to pass through the inside of the projecting part 32 and the base 31 from the conductive contact part 33 at the distal end of the projecting part 32 and to be exposed on the base upper surface 35 of the base 31.

For example, the distal end of the signal line part 34 may have either of a protruding structure, a substantially coplanar structure, or a buried structure with respect to the distal end portion of the projecting part 32 or the vicinity thereof, that is, the region where the conductive contact part 33 is formed. From the viewpoint of connection stability with the conductive contact part 33, a protruding structure may be used. A protruding portion at distal end of the signal line part 34 is partially or entirely covered with the conductive contact part 33. For the protruding structure at the distal end of the signal line part 34, it is possible to adopt a structure with or without folds or a structure wrapped around the surface of the distal end part of the projecting part 32. As another wiring structure of the signal line part 34, a structure provided on the surfaces of the projecting part 32 and the base 31 may be used, or a wiring structure in which a part is provided on the inside and a part is provided on the surface may be used.

### <Materials of Electrode Part Main Body 30 and Electrode Fixing Member 70>

Materials of the electrode part main body 30 (base 31 and projecting part 32) and the electrode fixing member 70 will be described. The electrode part main body 30 (the base 31 and the projecting part 32) and the electrode fixing member 70 may be made of the rubber-like elastic body as described above. Specifically, the rubber-like elastic body is rubber or a thermoplastic elastomer (also simply referred to as "elastomer (TPE)"). Examples of the rubber include silicone rubber. Examples of the thermoplastic elastomer include styrene-based TPE (TPS), olefin-based TPE (TPO), vinyl chloride-based TPE (TPVC), urethane-based TPE (TPU), ester-based TPE (TPEE), amide-based TPE (TPAE), and the like.

In a case where the electrode part main body 30 is made of silicone rubber, the rubber hardness A is, for example, 15 or more and 55 or less in a case where the type A durometer hardness on the surface of the electrode part main body 30 (the base 31 or the projecting part 32), which is measured at 37°C in accordance with JIS K 6253 (1997), is defined as the rubber hardness A.

Next, the aforementioned silicone rubber-based curable composition will be described.

The silicone rubber may be configured with a cured substance of the silicone rubber-based curable composition. A step of curing the silicone rubber-based curable resin composition is performed, for example, by heating the composition at 100°C to 250°C for 1 to 30 minutes (primary curing) and post-baking the composition at 100°C to 200°C for 1 to 4 hours (secondary curing).

Insulating silicone rubber is silicone rubber not containing a conductive filler, and conductive silicone rubber is silicone rubber containing a conductive filler.

The silicone rubber-based curable composition according to the present embodiment may contain a vinyl group-containing organopolysiloxane (A). The vinyl group-containing organopolysiloxane (A) is a polymer containing the silicone rubber-based curable composition according to the present embodiment as a main element.

An insulating silicone rubber-based curable composition and a conductive silicone rubber-based curable composition may contain the same type of vinyl group-containing linear organopolysiloxanes. The same type of vinyl group-containing linear organopolysiloxanes may contain at least vinyl groups having the same functional groups and may be linear. The amount of vinyl groups in the molecule, the molecular weight distribution, or the addition amounts thereof may vary between the vinyl group-containing linear organopolysiloxanes.

The insulating silicone rubber-based curable composition and the conductive silicone rubber-based curable composition may further contain different types of vinyl group-containing organopolysiloxanes.

The vinyl group-containing organopolysiloxane (A) can contain a vinyl group-containing linear organopolysiloxane (A1) having a linear structure.

The vinyl group-containing linear organopolysiloxane (A1) has a linear structure and contains a vinyl group which functions as a crosslinking point during curing.

The content of the vinyl group in the vinyl group-containing linear organopolysiloxane (A1) is not particularly limited. For example, the vinyl group in the vinyl group-containing linear organopolysiloxane (A1) has 2 or more vinyl groups in the molecule, and the content of the vinyl groups is preferably 15 mol% or less and more preferably 0.01 to 12 mol%. In a case where the content of the vinyl group is in the above range, the amount of the vinyl group in the vinyl group-containing linear organopolysiloxane (A1) can be optimized, and a network between the respective components, which will be described later, can be reliably formed. In the present embodiment, "to" means that the range includes numerical values as upper and lower limits.

In the present specification, the vinyl group content is mol% of a vinyl group-containing siloxane unit with respect to 100 mol% of all the units forming the vinyl group-containing linear organopolysiloxane (A1). Here, it is assumed that there may be one vinyl group for each vinyl group-containing siloxane unit.

The polymerization degree of the vinyl group-containing linear organopolysiloxane (A1) is not particularly limited and is, for example, preferably in a range of about 1,000 to 10,000 and more preferably in a range of about 2,000 to 5,000. The polymerization degree can be determined, for example, as a polystyrene-equivalent number-average polymerization degree (or number-average molecular weight) or the like gel permeation chromatography (GPC) using chloroform as an elution solvent.

The specific gravity of the vinyl group-containing linear organopolysiloxane (A1) is not particularly limited and is preferably in a range of about 0.9 to 1.1.

Using the vinyl group-containing linear organopolysiloxane (A1) having the polymerization degree and the specific gravity in the ranges described above makes it possible to obtain silicone rubber having higher heat resistance, higher flame retardancy, higher chemical stability, and the like.

It is preferable that the vinyl group-containing linear organopolysiloxane (A1) have a structure represented by the following Formula (1).

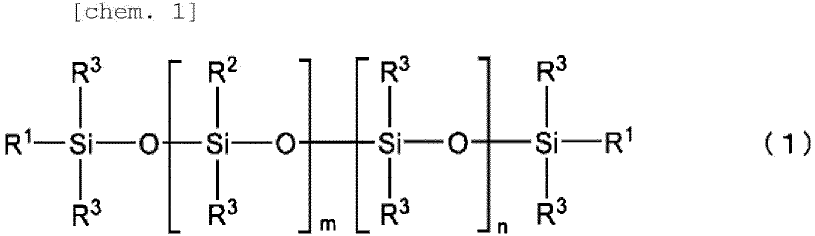

In Formula (1), R¹ is a substituted or unsubstituted alkyl group, alkenyl group, or aryl group having 1 to 10 carbon atoms, or a hydrocarbon group formed by the combination of these groups. Examples of the alkyl group having 1 to 10 carbon atoms include a methyl group, an ethyl group, a propyl group, and the like. Among these, a methyl group is preferable. Examples of the alkenyl group having 1 to 10 carbon atoms include a vinyl group, an allyl group, a butenyl group, and the like. Among these, a vinyl group is preferable. Examples of the aryl group having 1 to 10 carbon atoms include a phenyl group and the like.

In addition, R² represents a substituted or unsubstituted alkyl group, alkenyl group, or aryl group having 1 to 10 carbon atoms, or a hydrocarbon group including a combination thereof. Examples of the alkyl group having 1 to 10 carbon atoms include a methyl group, an ethyl group, a propyl group, and the like. Among these, a methyl group is preferable. Examples of the alkenyl group having 1 to 10 carbon atoms include a vinyl group, an allyl group, and a butenyl group. Examples of the aryl group having 1 to 10 carbon atoms include a phenyl group.

In addition, R³ is a substituted or unsubstituted alkyl group or aryl group having 1 to 8 carbon atoms, or a hydrocarbon group formed by the combination of these groups. Examples of the alkyl group having 1 to 8 carbon atoms include a methyl group, an ethyl group, a propyl group, and the like. Among these, a methyl group is preferable. Examples of the aryl group having 1 to 8 carbon atoms include a phenyl group.

Furthermore, examples of a substituent of R¹ and R² in Formula (1) include a methyl group and a vinyl group. Examples of a substituent of R³ include a methyl group.

In Formula (1), a plurality of R¹'s are independent of each other, and may be the same as or different from each other. Furthermore, this shall apply to R² and R³.

m and n each represent the number of repeating units configuring the vinyl group-containing linear organopolysiloxane (A1) represented by Formula (1). m represents an integer of 0 to 2,000, and n represents an integer of 1,000 to 10,000. m is preferably 0 to 1,000, and n is preferably 2,000 to 5,000.

Examples of specific structures of the vinyl group-containing linear organopolysiloxane (A1) represented by Formula (1) include a structure represented by the following Formula (1-1) .

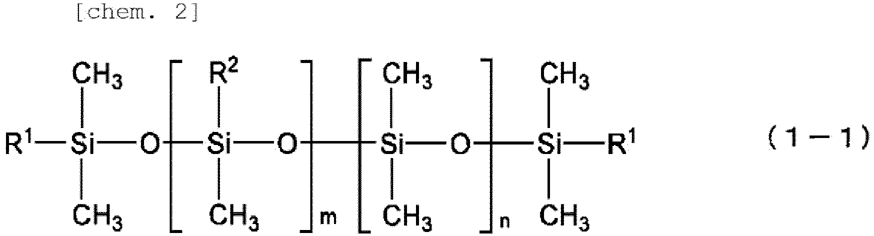

In Formula (1-1), R¹ and R² are each independently a methyl group or a vinyl group, and at least one of R¹ and R² is the vinyl group.

It is preferable that the vinyl group-containing linear organopolysiloxane (A1) contain a first vinyl group-containing linear organopolysiloxane (A1-1) that has two or more vinyl groups in the molecule and has a vinyl group content of 0.4 mol% or less and a second vinyl group-containing linear organopolysiloxane (A1-2) that has a vinyl group content of 0.5 to 15 mol%. Combining the first vinyl group-containing linear organopolysiloxane (A1-1) having the general vinyl group content and the second vinyl group-containing linear organopolysiloxane (A1-2) having a high vinyl group content as raw rubber that is a raw material of the silicone rubber enables vinyl groups to be dispersed, which makes it possible to more effectively create variations in the crosslinking density in the crosslinked network of the silicone rubber. As a result, the tear strength of the silicone rubber can be more effectively improved.

Specifically, as the vinyl group-containing linear organopolysiloxane (A1), for example, the first vinyl group-containing linear organopolysiloxane (A1-1) having two or more of a unit in which R¹ in Formula (1-1) represents a vinyl group and/or a unit in which R² in Formula (1-1) represents a vinyl group in the molecule and the content is 0.4 mol% or less, or the second vinyl group-containing linear organopolysiloxane (A1-2) including 0.5 to 15 mol% of a unit in which R¹ in Formula (1-1) represents a vinyl group and/or a unit in which R² in Formula (1-1) represents a vinyl group is preferably used.

The first vinyl group-containing linear organopolysiloxane (A1-1) preferably has a vinyl group content of 0.01 to 0.2 mol%. In addition, the second vinyl group-containing linear organopolysiloxane (A1-2) preferably has a vinyl group content of 0.8 to 12 mol%.

In a case where the first vinyl group-containing linear organopolysiloxane (A1-1) and the second vinyl group-containing linear organopolysiloxane (A1-2) are mixed together, the ratio between (A1-1) and (A1-2) is not particularly limited. For example, the weight ratio (A1-1):(A1-2) is preferably 50:50 to 95:5, and more preferably 80:20 to 90:10.

As each of the first and second vinyl group-containing linear organopolysiloxanes (A1-1) and (A1-2), only one compound may be used, or two or more compounds may be used in combination.

The vinyl group-containing organopolysiloxane (A) may contain a vinyl group-containing branched organopolysiloxane (A2) having a branched structure.

### <<Organohydrogen Polysiloxane (B)>>

The silicone rubber-based curable composition of the present embodiment may contain a crosslinking agent. The crosslinking agent can contain an organohydrogen polysiloxane (B) .

The organohydrogen polysiloxane (B) is classified into a linear organohydrogen polysiloxane (B1) having a linear structure and a branched organohydrogen polysiloxane (B2) having a branched structure, and can include either or both of these.

The insulating silicone rubber-based curable composition and the conductive silicone rubber-based curable composition may contain the same type of crosslinking agents. The same type of crosslinking agents just need to have at least a common structure such as a linear structure or a branched structure. The crosslinking agents may have different molecular weight distributions in the molecule or may contain different functional groups, and the addition amounts thereof may be different.

The insulating silicone rubber-based curable composition and the conductive silicone rubber-based curable composition may further contain different types of crosslinking agents.

The linear organohydrogen polysiloxane (B1) is a polymer that has a linear structure and a structure (=Si-H) in which hydrogen is directly bonded to Si, and has a hydrosilylation reaction with vinyl groups contained in the components mixed with the silicone rubber-based curable composition in addition to the vinyl groups of the vinyl group-containing organopolysiloxane (A) to crosslink the components.

The molecular weight of the linear organohydrogen polysiloxane (B1) is not particularly limited. For example, the weight-average molecular weight thereof is preferably 20,000 or less, and more preferably 1,000 or more and 10,000 or less.

The weight-average molecular weight of the linear organohydrogen polysiloxane (B1) can be measured as a polystyrene-equivalent value by gel permeation chromatography (GPC) using chloroform as an elution solvent.

It is preferable that the linear organohydrogen polysiloxane (B1) do not have a vinyl group in general. In a case where the linear organohydrogen polysiloxane (B1) does not have a vinyl group, it is possible to reliably prevent the occurrence of a crosslinking reaction in the molecule of the linear organohydrogen polysiloxane (B2).

As the linear organohydrogen polysiloxane (B1), for example, a compound having a structure represented by the following Formula (2) is preferably used.

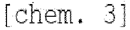

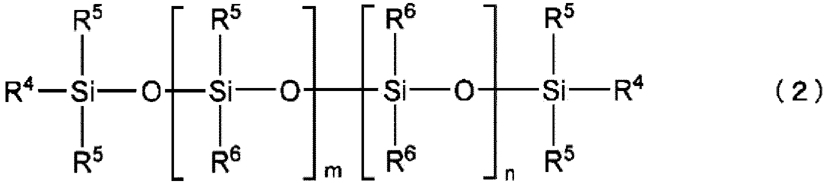

In Formula (2), R⁴ is a substituted or unsubstituted alkyl group, alkenyl group, or aryl group having 1 to 10 carbon atoms, a hydrocarbon group formed by the combination of these groups, or a hydride group. Examples of the alkyl group having 1 to 10 carbon atoms include a methyl group, an ethyl group, a propyl group, and the like. Among these, a methyl group is preferable. Examples of the alkenyl group having 1 to 10 carbon atoms include a vinyl group, an allyl group, a butenyl group, and the like. Examples of the aryl group having 1 to 10 carbon atoms include a phenyl group.

In addition, R⁵ is a substituted or unsubstituted alkyl group, alkenyl group, or aryl group having 1 to 10 carbon atoms, a hydrocarbon group formed by the combination of these groups, or a hydride group. Examples of the alkyl group having 1 to 10 carbon atoms include a methyl group, an ethyl group, and a propyl group. Among these, a methyl group is preferable. Examples of the alkenyl group having 1 to 10 carbon atoms include a vinyl group, an allyl group, a butenyl group, and the like. Examples of the aryl group having 1 to 10 carbon atoms include a phenyl group.

In Formula (2), a plurality of R⁴'s are independent of each other, and may be the same as or different from each other.

This shall apply to R⁵. In this case, at least two or more among a plurality of R⁴'s and a plurality of R⁵'s represent a hydride group.

In addition, R⁶ is a substituted or unsubstituted alkyl group or aryl group having 1 to 8 carbon atoms, or a hydrocarbon group formed by the combination of these groups. Examples of the alkyl group having 1 to 8 carbon atoms include a methyl group, an ethyl group, a propyl group, and the like. Among these, a methyl group is preferable. Examples of the aryl group having 1 to 8 carbon atoms include a phenyl group. A plurality of R⁶'s may be independent from each other and may be the same as or different from each other.

Examples of the substituent of R⁴, R⁵, and R⁶ in Formula (2) include a methyl group and a vinyl group, and the methyl group is preferable from the viewpoint that the crosslinking reaction in the molecule is prevented.

m and n each independently represent the number of repeating units configuring the linear organohydrogen polysiloxane (B1) represented by Formula (2), m represents an integer of 2 to 150, and n represents an integer of 2 to 150. It is preferable that m represent an integer of 2 to 100 and n represent an integer of 2 to 100.

As the linear organohydrogen polysiloxane (B1), only one compound may be used alone, or two or more compounds may be used in combination.

Having a branched structure, the branched organohydrogen polysiloxane (B2) is a component that largely contributes to the formation of a density-varying crosslinking structure in the silicone rubber system by forming a region having a high crosslinking density. In addition, just as the linear organohydrogen polysiloxane (B1), the branched organohydrogen polysiloxane (B2) is a polymer that has a structure (=Si-H) in which hydrogen is directly bonded to Si, and has a hydrosilylation reaction with vinyl groups of components mixed with the silicone rubber-based curable composition in addition to the vinyl groups of the vinyl group-containing organopolysiloxane (A) to crosslink these components.

The specific gravity of the branched organohydrogen polysiloxane (B2) is in a range of 0.9 to 0.95.

It is preferable that the branched organohydrogen polysiloxane (B2) do not have a vinyl group in general. In a case where the branched organohydrogen polysiloxane (B2) does not have a vinyl group, it is possible to reliably prevent the occurrence of a crosslinking reaction in the molecule of the linear organohydrogen polysiloxane (B2).

The branched organohydrogen polysiloxane (B2) is preferably represented by the following Average Compositional Formula (c).

Average Compositional Formula (c) (Hₐ(R⁷)₃₋ₐSiO_{1/2})ₘ(SiO_{4/2})ₙ

(In Formula (c), R⁷ represents a monovalent organic group, a represents an integer of 1 to 3, m represents the number of Hₐ(R⁷)₃₋ₐSiO_{1/2} units, and n represents the number of SiO_{4/2} units.)

In Formula (c), R⁷ is a monovalent organic group, preferably a substituted or unsubstituted alkyl group or aryl group having 1 to 10 carbon atoms, or a hydrocarbon group formed by the combination of these groups. Examples of the alkyl group having 1 to 10 carbon atoms include a methyl group, an ethyl group, a propyl group, and the like. Among these, a methyl group is preferable. Examples of the aryl group having 1 to 10 carbon atoms include a phenyl group.

In Formula (c), a represents the number of hydride groups (hydrogen atoms directly bonded to Si) which is an integer of 1 to 3. a is preferably 1.

In addition, in Formula (c), m is the number of Hₐ(R⁷)₃₋ₐSiO_{1/2} units, and n is the number of SiO_{4/2} units.

The branched organohydrogen polysiloxane (B2) has a branched structure. The difference between the linear organohydrogen polysiloxane (B1) and the branched organohydrogen polysiloxane (B2) is whether the structure thereof is linear or branched. Assuming that the number of Si is 1, the number of alkyl groups R bonded to Si (R/Si) is in a range of 1.8 to 2.1 in the linear organohydrogen polysiloxane (B1) and is in a range of 0.8 to 1.7 in the branched organohydrogen polysiloxane (B2).

Having a branched structure, the branched organohydrogen polysiloxane (B2) leaves 5% or more residues in a case where the compound is heated, for example, to 1,000°C at a heating rate of 10 °C/min in a nitrogen atmosphere. On the other hand, having a linear structure, the linear organohydrogen polysiloxane (B1) substantially does not leave residues in a case where the compound is heated under the above conditions.

Specific examples of the branched organohydrogen polysiloxane (B2) include compounds having a structure represented by the following Formula (3).

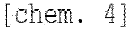

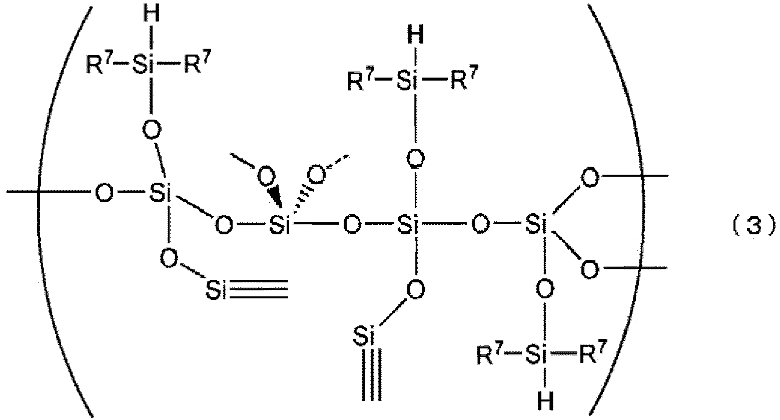

In Formula (3), R⁷ is a substituted or unsubstituted alkyl group or aryl group having 1 to 8 carbon atoms, or a hydrocarbon group formed by the combination of these groups, or a hydrogen atom. Examples of the alkyl group having 1 to 8 carbon atoms include a methyl group, an ethyl group, a propyl group, and the like. Among these, a methyl group is preferable. Examples of the aryl group having 1 to 8 carbon atoms include a phenyl group. Examples of the substituent of R⁷ include a methyl group.

In Formula (3), a plurality of R⁷'s are independent of each other, and may be the same as or different from each other.

In Formula (3), "-O-Si≡" means that Si has a branched structure that expands three-dimensionally.

As the branched organohydrogen polysiloxane (B2), only one compound may be used alone, or two or more compounds may be used in combination.

In each of the linear organohydrogen polysiloxane (B1) and the branched organohydrogen polysiloxane (B2), the amount of hydrogen atoms (hydride groups) directly bonded to Si is not particularly limited. Here, in the silicone rubber-based curable composition, the total amount of hydride groups in the linear organohydrogen polysiloxane (B1) and the branched organohydrogen polysiloxane (B2) is preferably 0.5 to 5 mol and more preferably 1 to 3.5 mol with respect to 1 mol of vinyl groups in the vinyl group-containing linear organopolysiloxane (A1). In a case where the total amount of hydride groups is in the above range, a crosslinked network can be reliably formed among the linear organohydrogen polysiloxane (B1) and the branched organohydrogen polysiloxane (B2), and the vinyl group-containing linear organopolysiloxane (A1).

### <<Silica Particles (C)>>

The silicone rubber-based curable composition of the present embodiment contains a non-conductive filler. As necessary, the non-conductive filler may contain silica particles (C). In a case where the silicone rubber-based curable composition contains the silica particles (C), the hardness or mechanical strength of the elastomer can be improved.

The insulating silicone rubber-based curable composition and the conductive silicone rubber-based curable composition may contain the same type of non-conductive fillers. The same type of non-conductive fillers just need to have at least a common constituent material, and the particle size, specific surface area, surface treatment agent, or the addition amount thereof may vary between the fillers.

The insulating silicone rubber-based curable composition and the conductive silicone rubber-based curable composition may further contain different types of silane coupling agents.

The silica particles (C) are not particularly limited. For example, fumed silica, sintered silica, precipitated silica, or the like is used. Each of these compounds may be used alone, or two or more compounds described above may be used in combination.

The specific surface area of the silica particles (C) measured using, for example, a BET method is, for example, preferably 50 to 400 m²/g and more preferably 100 to 400 m²/g. The average primary particle size of the silica particles (C) is, for example, preferably 1 to 100 nm and more preferably about 5 to 20 nm.

Using the silica particles (C) having the specific surface area and the average particle size in the above ranges makes it possible to improve the hardness and the mechanical strength of the formed silicone rubber and, particularly, to improve the tensile strength of the formed silicone rubber.

### <<Silane Coupling Agent (D)>>

The silicone rubber-based curable composition according to the present embodiment may contain a silane coupling agent (D).

The silane coupling agent (D) may have a hydrolyzable group. The hydrolyzable group is hydrolyzed into a hydroxyl group by water. The hydroxyl group has a dehydration condensation reaction with a hydroxyl group on the surface of the silica particles (C), which can modify the surface of the silica particles (C).

The insulating silicone rubber-based curable composition and the conductive silicone rubber-based curable composition may contain the same type of silane coupling agents. The same type of silane coupling agents just need to have at least a common functional group and may have other functional groups in the molecule, and the addition amounts thereof may be different.

The insulating silicone rubber-based curable composition and the conductive silicone rubber-based curable composition may further contain different types of silane coupling agents.

The silane coupling agent (D) can include a silane coupling agent having a hydrophobic group. In this case, the hydrophobic group is added to the surfaces of the silica particles (C). Accordingly, in the silicone rubber-based curable composition and the silicone rubber, the cohesive force between the silica particles (C) decreases (cohesion through a hydrogen bond formed by a silanol group weakens). Presumably, as a result, the dispersibility of the silica particles (C) in the silicone rubber-based curable composition may be improved. Consequently, the interface between the silica particles (C) and a rubber matrix enlarges, and a reinforcing effect of the silica particles (C) increases. Furthermore, presumably, the silica particles (C) in the matrix may become more slippery during the matrix deformation of rubber. The improvement of dispersibility and slipperiness of the silica particles (C) leads to the improvement of the mechanical strength (for example, tensile strength, tear strength, or the like) of the silicone rubber by the silica particles (C).

The silane coupling agent (D) may include a silane coupling agent having a vinyl group. In a case where the silane coupling agent has a vinyl group, the vinyl group is introduced into the surface of the silica particles (C). Therefore, in a case where the silicone rubber-based curable composition is cured, that is, in a case where the vinyl groups of the vinyl group-containing organopolysiloxane (A) and the hydride groups in the organohydrogen polysiloxane (B) have a hydrosilylation reaction to form a network (crosslinked structure), the vinyl groups of the silica particles (C) are also involved in the hydrosilylation reaction with the hydride groups of the organohydrogen polysiloxane (B). As a result, the silica particles (C) are also incorporated into the network. Accordingly, it is possible to achieve the reduction in hardness and the increase in modulus of the formed silicone rubber.

As the silane coupling agent (D), a silane coupling agent having a hydrophobic group and a silane coupling agent having a vinyl group may be used in combination.

Examples of the silane coupling agent (D) include a compound represented by the following Formula (4).

Yₙ-Si-(X)₄₋ₙ ... (4)

In Formula (4), n represents an integer of 1 to 3. Y represents any functional group among a hydrophobic group, a hydrophilic group, and a vinyl group. When n represents 1, Y represents a hydrophobic group. When n represents 2 or 3, at least one of Y's represents a hydrophobic group. X represents a hydrolyzable group.

The hydrophobic group is an alkyl group or aryl group having 1 to 6 carbon atoms, or a hydrocarbon group as a combination of these. Examples of the hydrophobic group include a methyl group, an ethyl group, a propyl group, a phenyl group, and the like. Among these, a methyl group is particularly preferable.

Examples of the hydrophilic group include a hydroxyl group, a sulfonate group, a carboxyl group, a carbonyl group, and the like. Among these, a hydroxyl group is particularly preferable. The hydrophilic group may be incorporated into the silane coupling agent as a functional group. From the viewpoint of making the silane coupling agent (D) hydrophobic, it is preferable that the silane coupling agent (D) do not contain a hydrophilic group.

Examples of the hydrolyzable group include an alkoxy group such as a methoxy group or an ethoxy group, a chloro group, a silazane group, and the like. Among these, in view of high reactivity with the silica particles (C), a silazane group is preferable. The silane coupling agent (D) having a silazane group as the hydrolyzable group has a structure including two structures represented by (Yₙ-Si-) in Formula (4).

Specific examples of the silane coupling agent (D) represented by Formula (4) are as follows.

Examples of the silane coupling agent having a hydrophobic group as the functional group include an alkoxysilane such as methyltrimethoxysilane, dimethyldimethoxysilane, phenyltrimethoxysilane, methyltriethoxysilane, dimethyldiethoxysilane, phenyltriethoxysilane, n-propyltrimethoxysilane, n-propyltriethoxysilane, hexyltrimethoxysilane, hexyltriethoxysilane, or decyltrimethoxysilane; a chlorosilane such as methyltrichlorosilane, dimethyldichlorosilane, trimethylchlorosilane, or phenyltrichlorosilane; and hexamethyldisilazane. Among these, a silane coupling agent having a trimethylsilyl group that includes one or more compounds selected from the group consisting of hexamethyldisilazane, trimethylchlorosilane, trimethylmethoxysilane, and trimethylethoxysilane is preferable.

Examples of the silane coupling agent having a vinyl group as the functional group include an alkoxysilane such as methacryloxypropyltriethoxysilane, methacryloxypropyltrimethoxysilane, methacryloxypropylmethyldiethoxysilane, methacryloxypropylmethyldimethoxysilane, vinyltriethoxysilane, vinyltrimethoxysilane, or vinylmethyldimethoxysilane; a chlorosilane such as vinyltrichlorosilane or vinylmethyldichlorosilane; and divinyltetramethyldisilazane. Among these, a silane coupling agent having a vinyl group-containing organosilyl group that includes one or more compounds selected from the group consisting of methacryloxypropyltriethoxysilane, methacryloxypropyltrimethoxysilane, methacryloxypropylmethyldiethoxysilane, methacryloxypropylmethyldimethoxysilane, divinyltetramethyldisilazane, vinyltriethoxysilane, vinyltrimethoxysilane, and vinylmethyldimethoxysilane is preferable.

In a case where the silane coupling agent (D) includes two silane coupling agents, a silane coupling agent having a trimethylsilyl group and a silane coupling agent having a vinyl group-containing organosilyl group, it is preferable that the silane coupling agent (D) include hexamethyldisilazane as a silane coupling agent having a hydrophobic group and divinyltetramethyldisilazane as a silane coupling agent having a vinyl group.

In a case where a silane coupling agent (D1) having a trimethylsilyl group and a silane coupling agent (D2) having a vinyl group-containing organosilyl group are used in combination, a ratio between (D1) and (D2) is not particularly limited. For example, a weight ratio (D1):(D2) is 1:0.001 to 1:0.35, preferably 1:0.01 to 1:0.20, and more preferably 1:0.03 to 1:0.15. Setting the ratio to the numerical range described above makes it possible to obtain desired physical properties of silicone rubber. Specifically, the dispersibility of silica in the rubber and the crosslinking properties of the rubber can be balanced.

In the present embodiment, the lower limit of the content of the silane coupling agent (D) with respect to the total amount of 100 parts by weight of the vinyl group-containing organopolysiloxane (A) is preferably 1% by mass or more, more preferably 3% by mass or more, and even more preferably 5° by mass or more. Furthermore, the upper limit of the content of the silane coupling agent (D) with respect to the total amount of 100 parts by weight of the vinyl group-containing organopolysiloxane (A) is preferably 100% by mass or less, more preferably 80% by mass or less, and even more preferably 40% by mass or less.

Setting the content of the silane coupling agent (D) to be equal to or more than the aforementioned lower limit makes it possible to improve the adhesion between a columnar portion containing an elastomer and the conductive resin layer. In addition, the mechanical strength of the silicone rubber can be improved. Furthermore, setting the content of the silane coupling agent (D) to be equal to or less than the aforementioned upper limit makes it possible to obtain silicone rubber having appropriate mechanical characteristics.

### <<Platinum or Platinum Compound (E)>>

The silicone rubber-based curable composition according to the present embodiment may contain a catalyst. The catalyst can include platinum or platinum compound (E). The platinum or platinum compound (E) is a catalyst component that functions as a catalyst during curing. The addition amount of the platinum or platinum compound (E) is the amount of the catalyst.

The insulating silicone rubber-based curable composition and the conductive silicone rubber-based curable composition may contain the same type of catalysts. The same type of catalysts just need to have at least a common constituent material. Different compositions may be contained in the catalysts, or the addition amounts thereof may vary between the catalysts.

The insulating silicone rubber-based curable composition and the conductive silicone rubber-based curable composition may further contain different catalysts.

As the platinum or platinum compound (E), known platinum or platinum compounds can be used. Examples thereof include platinum black, silica or carbon black on which platinum is supported, platinic chloride or an alcohol solution of platinic chloride, a complex salt of platinic chloride and olefin, a complex salt of platinic chloride and vinylsilxoane, and the like.

As the platinum or platinum compound (E), only one platinum element or one platinum compound may be used alone, or two or more platinum elements or two platinum compounds may be used in combination.

In the present embodiment, the content of the platinum or platinum compound (E) in the silicone rubber-based curable composition refers to the amount of the catalyst and may be appropriately set. Specifically, the content of platinum group metals by weight with respect to the total amount of 100 parts by weight of the vinyl group-containing organopolysiloxane (A), the silica particles (C), and the silane coupling agent (D) is 0.01 to 1000 ppm and preferably 0.1 to 500 ppm.

Setting the content of the platinum or platinum compound (E) to be equal to or more than the aforementioned lower limit makes it possible to cure the silicone rubber-based curable composition at an appropriate rate. In addition, setting the content of the platinum or platinum compound (E) to be equal to or less than the aforementioned upper limit makes it possible to reduce manufacturing costs.

### <<Water (F)>>

The silicone rubber-based curable composition according to the present embodiment may contain water (F) in addition to the components (A) to (E).

The water (F) is a component that functions as a dispersion medium for dispersing the respective components in the silicone rubber-based curable composition and contributes to the reaction between the silica particles (C) and the silane coupling agent (D). Therefore, the silica particles (C) and the silane coupling agent (D) can be more reliably linked to each other in the silicone rubber, and the silicone rubber can show uniform characteristics as a whole.

### (Other components)

Further, the silicone rubber-based curable composition according to the present embodiment may further contain other components in addition to the components (A) to (F). Examples of those other components include an inorganic filler other than the silica particles (C), such as diatomaceous earth, iron oxide, zinc oxide, titanium oxide, barium oxide, magnesium oxide, cerium oxide, calcium carbonate, magnesium carbonate, zinc carbonate, glass wool, or mica, and an additive such as a reaction inhibitor, a dispersant, a pigment, a dye, an antistatic agent, an antioxidant, a flame retardant, or a thermal conductivity enhancing agent.

The conductive solution (conductive silicone rubber composition) according to the present embodiment contains the aforementioned conductive filler and a solvent in addition to the silicone rubber-based curable composition that does not contain a conductive filler.

As the solvent, various known solvents can be used. For example, the solvent can include a high boiling point solvent. Each of these compounds may be used alone, or two or more compounds described above may be used in combination.

Examples of the solvent include aliphatic hydrocarbons such as pentane, hexane, cyclohexane, heptane, methylcyclohexane, ethylcyclohexane, octane, decane, dodecane, and tetradecane; aromatic hydrocarbons such as benzene, toluene, ethylbenzene, xylene, trifluoromethylbenzene, and benzotrifluoride; ethers such as diethyl ether, diisopropyl ether, dibutyl ether, cyclopentyl methyl ether, cyclopentyl ethyl ether, ethylene glycol dimethyl ether, ethylene glycol diethyl ether, diethylene glycol dimethyl ether, 1,4-dioxane, 1,3-dioxane, and tetrahydrofuran; haloalkanes such as dichloromethane, chloroform, 1,1-dichloroethane, 1,2-dichloroethane, 1,1,1-trichloroethane, and 1,1,2-trichloroethane; carboxylic acid amids such as N,N-dimethylformamide and N,N-dimethylacetamide; sulfoxides such as dimethyl sulfoxide and diethyl sulfoxide, and the like. Each of these compounds may be used alone, or two or more compounds described above may be used in combination.

Adjusting the solid content or the like of the conductive solution enables the solution to have viscosity appropriate for various coating methods such as spray coating and dip coating.

In a case where the conductive solution contains the conductive filler and the silica particles (C), the lower limit of the content of the silica particles (C) contained in the electrode part main body 30 with respect to the total amount of 100% by mass of the silica particles (C) and the conductive filler is, for example, 1% by mass or more, preferably 3% by mass or more, and more preferably 5° by mass or more. In a case where the lower limit is in the above range, the mechanical strength of the electrode part main body 30 can be improved. On the other hand, the upper limit of the content of the silica particles (C) contained in the electrode part main body 30 with respect to the total amount of 100% by mass of the silica particles (C) and the conductive filler is, for example, 20% by mass or less, preferably 15% by mass or less, and more preferably 10% by mass or less. In a case where the upper limit is in the above range, conductivity and mechanical strength or flexibility in the electrode part main body 30 can be balanced.

By heating and drying the conductive solution as necessary, the conductive silicone rubber is obtained.

The conductive silicone rubber may be configured such that it does not contain silicone oil. In a case where the conductive silicone rubber is configured as above, it is possible to prevent a phenomenon where silicone oil bleeds out to the surface of the electrode part main body 30 and reduces conductivity.

### <Material of Signal Line Part 34>

As the signal line part 34, known materials can be used. For example, the signal line part 34 can be configured with conductive fiber. As the conductive fiber, it is possible to use one or more fibers selected from the group consisting of metal fiber, metal-coated fiber, carbon fiber, conductive polymer fiber, conductive polymer-coated fiber, and conductive paste-coated fiber. Each of these compounds may be used alone, or two or more compounds described above may be used in combination.

The metal material of the metal fiber and the metal-coated fiber is not particularly limited as long as it has conductivity. Examples of the metal material include copper, silver, gold, nickel, tin, lead, zinc, bismuth, antimony, stainless steel, aluminum, silver/silver chloride, and alloys of these. Each of these compounds may be used alone, or two or more compounds described above may be used in combination. Among these, from the viewpoint of conductivity, silver can be used. In addition, it is preferable that the metal material do not include a metal such as chromium that imposes burden on the environment.

The fiber material of the metal-coated fiber, the conductive polymer-coated fiber, and the conductive paste-coated fiber is not particularly limited, and may be any of synthetic fiber, semisynthetic fiber, and natural fiber. Among these, polyester, nylon, polyurethane, silk, cotton, and the like are preferably used. Each of these compounds may be used alone, or two or more compounds described above may be used in combination.

Examples of the carbon fiber include a PAN-based carbon fiber and a pitch-based carbon fiber.

As the conductive polymer material of the conductive polymer fiber and the conductive polymer-coated fiber, for example, polythiophene, polypyrrole, polyaniline, polyacetylene, polyphenylene vinylene, polynaphthalene, a mixture of conductive polymers of derivatives of these and a binder resin, or an aqueous solution of a conductive polymer such as PEDOT-PSS ((3,4-ethylenedioxythiophene)-poly(styrene sulfonate)) is used.

The resin material contained in the conductive paste of the conductive paste-coated fiber is not particularly limited, and preferably has elasticity. For example, the resin material includes one or more materials selected from the group consisting of silicone rubber, urethane rubber, fluororubber, nitrile rubber, acrylic rubber, styrene rubber, chloroprene rubber, and ethylene propylene rubber. Each of these compounds may be used alone, or two or more compounds described above may be used in combination.

The conductive filler contained in the conductive paste of the conductive paste-coated fiber is not particularly limited, and a known conductive material may be used. For example, the conductive filler can include one or more materials selected from the group consisting of metal particles, metal fiber, metal-coated fiber, carbon black, acetylene black, graphite, carbon fiber, carbon nanotubes, a conductive polymer, conductive polymer-coated fiber, and metal nanowires.

The metal configuring the conductive filler is not particularly limited. For example, the metal may include at least one metal or two or more metals among copper, silver, gold, nickel, tin, lead, zinc, bismuth, antimony, silver/silver chloride, and alloys of these. Among these, in view of high conductivity and high availability, silver or copper is preferable.

The signal line part 34 may be configured with spun yarn obtained by twisting a plurality of linear conductive fibers. Using such spun yarn makes it possible to suppress disconnection of the signal line part 34 during deformation.

In the present embodiment, coating of conductive fiber means not only covering of the outer surface of the fiber material. In the case of spun yarn obtained by twisting single fibers, the coating of conductive fiber also means that the voids of the fiber in the spun yarn are impregnated with a metal, a conductive polymer, or a conductive paste such that each of the single fibers configuring the spun yarn is coated.

The tensile elongation at break of the signal line part 34 is, for example, 1% or more and 50% or less, and preferably 1.5% or more and 45%. In a case where the tensile elongation at break is in such a numerical range, it is possible to inhibit the projecting parts 32 from being excessively deformed while suppressing break during deformation.

### <Material of Conductive Contact Part 33>

The conductive member of the conductive contact part 33 is, for example, a paste containing a good conductive metal (so-called conductive paste). The good conductive metal includes one or more metals selected from the group consisting of copper, silver, gold, nickel, tin, lead, zinc, bismuth, antimony, and alloys of these. Particularly, from the viewpoint of availability and conductivity, silver, silver chloride, and copper are suitable.

In a case where the conductive contact part 33 is formed by using a paste containing a good conductive metal, the top of the projecting parts 32 formed of a rubber-like elastic body is dipped into a paste-formed conductive solution containing a good conductive metal (dip coating). As a result, the conductive contact part 33 is formed on the surface of the projecting part 32.

The conductive contact part 33 as a conductive resin layer may be formed by applying the conductive solution containing a conductive filler and a solvent to the projecting part 32. In this case, by using the material (silicone rubber) in the same series as the projecting part 32 as the solvent, the adhesion of the conductive contact part 33 (conductive resin layer) can be improved.

By heating and drying the conductive solution as necessary, the conductive silicone rubber is obtained.

The conductive silicone rubber may be configured such that it does not contain silicone oil. As a result, the silicone oil bleeds out to a surface of the conductive contact part 33 such that a decrease in conductivity can be suppressed.

As a result, it is possible to improve the pushing hair aside performance in a case where the brain wave measuring device 1 is mounted on the head 99. In addition, it is possible to sufficiently ensure the contact area of the conductive contact part 33 in a case where the brain wave measuring device 1 is mounted.

### <Cap-integrated Type Snap Button 50>

The cap-integrated type snap button 50 is made of, for example, a metal of the good conductor, has a bottomed cylindrical shape, and has a connection unit (male type snap button 53) for connection to an external measurement unit on an outer surface of a bottom portion. As the metal of the good conductor, for example, stainless steel, a copper alloy, an aluminum alloy, brass, or the like can be used.

The cap-integrated type snap button 50 integrally includes a cap top plate 51 which is a bottom surface having a bottomed cylindrical shape, and a cap barrel part 52 which has a tubular shape and extends out downward in the diagram from a peripheral edge of the cap top plate 51.

A male type snap button 53 having a columnar shape extending out upward in the diagram is provided at the center of the outer surface 56 of the circular cap top plate 51 (that is, the center of the circular shape in the top view). The base upper surface 35 of the electrode part main body 30 is attached to the inner surface 54 of the cap top plate 51 via the conductive paste layer to abut on the inner surface 54. At this time, the upper side end part of the signal line part 34 is arranged to be sandwiched between the inner surface 54 of the cap top plate 51 and the base upper surface 35 as described above.

The male type snap button 53 is an interface to which a signal line for connection to an external measurement unit is attached. Here, the male type snap button 53 has, for example, a shape of a connection terminal in which a distal end side having a columnar shape has a larger diameter than a root side. The male type snap button 53 is inserted through the through-hole 76 from the inside of the electrode fixing member 70 (electrode attachment member 75) and is fitted with the female type snap button 49 provided on the signal line at a portion having a larger diameter outside of the through-hole 76. At this time, the electrode attachment member 75 is sandwiched between the female type snap button 49 and the cap top plate 51, and the electrode part 10 is fixed to the electrode fixing member 70 with the projecting part 32 facing inward (toward the head 99 side).

According to the brain wave measuring device 1 of the present embodiment, since the front peripheral band member 21 of the frame 20 expands and contracts, the frame 20 can be easily mounted to the head circumference. In addition, since the electrode fixing member 70 to which the electrode part 10 is attached is provided to cover the occipital region, all the electrode parts 10 can comes into contact with the head 99 with an appropriate force in a case where the brain wave measuring device 1 is mounted. That is, the time for appropriately abutting the electrode part 10 on the head 99 can be shortened.

Although the embodiments of the present invention have been described with reference to the drawings, these are merely examples of the present invention, and various embodiments (modification examples) other than those described above can also be adopted. Such a modification example will be described below.

### <Modification Example of Electrode Fixing Member 70>

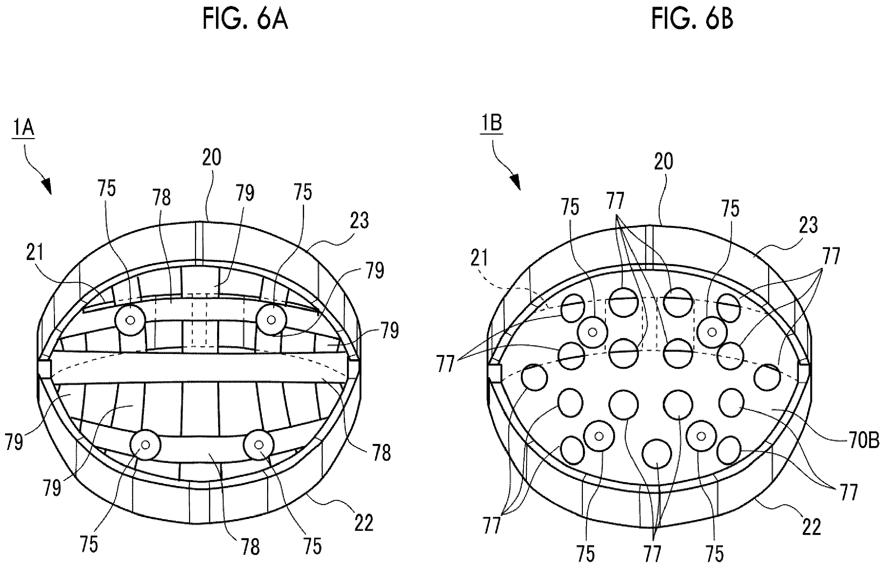

Figs. 6A and 6B show the brain wave measuring devices 1A and 1B having electrode fixing members 70A and 70B of the modification examples.

In the brain wave measuring device 1A of Fig. 6A, the electrode fixing member 70A is configured to have an elastic band member. Specifically, a plurality of band members (horizontal band members 78) provided to extend in the horizontal direction (that is, from one temporal region side to the other temporal region side) and a plurality of band members (vertical band members 79) provided to extend in the vertical direction (that is, direction from the occipital region toward the parietal region) are contained in the opening 25 on the occipital region side, which is formed by the rear peripheral frame 22 and the top frame 23, and intersect with each other in a lattice shape. As a result, the air permeability can be improved.

All or a part of the horizontal band members 78 and the vertical band members 79 may be attachable to and detachable or exchangeable from the rear peripheral frame 22 or the top frame 23. In addition, the brain wave measuring device 1 may be used in a state in which a part of the horizontal band members 78 or the vertical band members 79 is removed.

In the brain wave measuring device 1B of Fig. 6B, a plurality of through-holes 71 are provided in the elastic sheet member in the electrode fixing member 70B. As a result, the air permeability during the mounting can be improved. A size of the through-hole 71 is not particularly limited, and all the through-holes 71 may have the same size or may have different sizes.

### <Modification Example of Front Peripheral Band Member 21A>

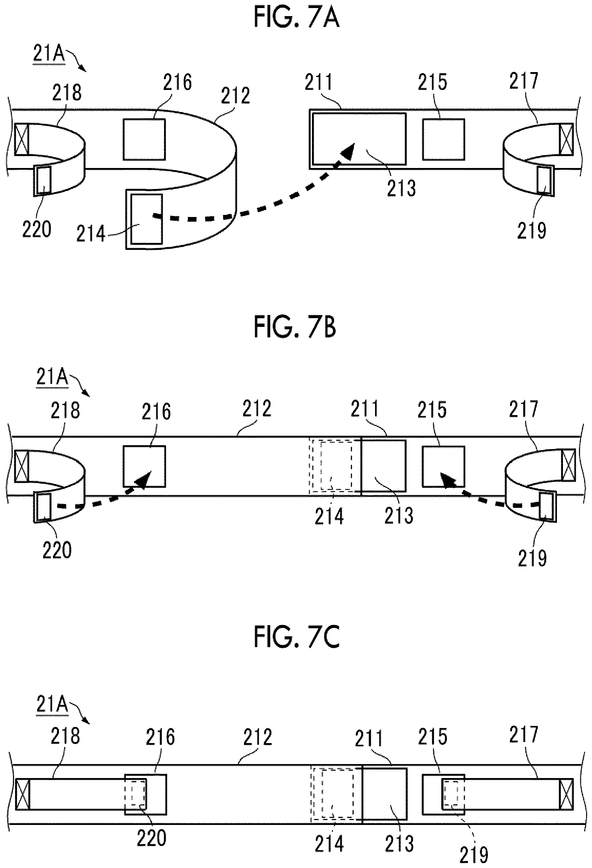

Figs. 7A, 7B, and 7C show a front peripheral band member 21A of the modification example. The front peripheral band member 21A is provided with a fixing mechanism including an elastic member and a hook and loop fastener in two stages as a configuration to function as a head circumference mounting adjusting part 91 that adjusts the mounting state in the head circumference direction.

The first-stage fixing mechanism is as follows. That is, as shown in Fig. 7A, the elastic band-shaped member extending in the head circumference direction is provided in the front peripheral band member 21A (head circumference mounting adjusting part 91) to be separable to the right and left. Specifically, the front peripheral band member 21A has a first band-shaped member 211 (right side in the drawing) and a second band-shaped member 212 (left side in the drawing), which are divided in the head circumference direction. The first band-shaped member 211 and the second band-shaped member 212 are provided to be bondable to each other to be adjustable in the length in the extending direction. More specifically, a hook and loop fastener (hook surface) 213 is provided on the distal end portion (the end part on the left side in the drawing) of the first band-shaped member 211 toward the outside. A hook and loop fastener (loop surface) 214 is provided on a distal end portion (an end part on the right side in the drawing) of the second band-shaped member 212 toward the inside. As shown in Fig. 7B, the hook and loop fastener (hook surface) 213 and the hook and loop fastener (loop surface) 214 are adhered to each other.

As a second-stage fixing mechanism, an auxiliary adjusting part 230 that acts to contract the elastic member of the head circumference mounting adjusting part 91 is provided.

Specifically, as a mechanism for adjusting the expanding state of the first band-shaped member 212, a first auxiliary band-shaped member 217 having an elastic band shape with one end fixed to the attachment part 221 is provided. The other end side of the first auxiliary band-shaped member 217 extends to the distal end side (left side in the drawing) of the first band-shaped member 211. A first auxiliary hook and loop fastener (hook surface) 215 is provided in a region on the right side of the hook and loop fastener (hook surface) 213 of the first band-shaped member 211 toward the outside. A first auxiliary hook and loop fastener (loop surface) 219 is provided on the other end side of the first auxiliary band-shaped member 217 toward the inside as a member adhered to the first auxiliary hook and loop fastener (hook surface) 215. As shown in Fig. 7C, the expanding state of the first auxiliary band-shaped member 217 can be adjusted by adjusting the adhesive position of the first auxiliary hook and loop fastener (loop surface) 219 with respect to the first auxiliary hook and loop fastener (hook surface) 215.

As a mechanism for adjusting the expanding state of the second band-shaped member 212, a second auxiliary band-shaped member 218 having an elastic band shape with one end fixed to the attachment part 222 is provided. The other end side of the second auxiliary band-shaped member 218 extends to the distal end side (right side in the drawing) of the second band-shaped member 212. A second auxiliary hook and loop fastener (hook surface) 216 is provided in a region on the left side of the hook and loop fastener (loop surface) 214 of the second band-shaped member 212 toward the outside. A second auxiliary hook and loop fastener (loop surface) 220 is provided on the other end side of the second auxiliary band-shaped member 218 toward the inside as a member adhered to the second auxiliary hook and loop fastener (hook surface) 216. As shown in Fig. 7C, the expanding state of the second auxiliary band-shaped member 218 can be adjusted by adjusting the adhesive position of the second auxiliary hook and loop fastener (loop surface) 220 with respect to the second auxiliary hook and loop fastener (hook surface) 216.

### <Another Embodiment of Mounting Adjusting Part>

As a mechanism for adjusting the mounting state of the brain wave measuring device 1 on the head 99, a vertex mounting adjusting part that adjusts the mounting state in the vertex direction may be provided. As the vertex mounting adjusting part, a band-shaped member or a string-shaped member that tightens the jaw part of the subject is used. Specifically, as a configuration of the vertex mounting adjusting part in which both ends of a band-shaped member or a string-like member, which is expandable and contractible and is rubber-like, are attached to two connecting parts 29 of the frame 20, the vertex mounting adjusting part is hooked on the chin during the mounting of the brain wave measuring device 1. In addition, the vertex mounting adjusting part may have an adjustment function by a hook and loop fastener mechanism, as in the front peripheral band member 21A as shown in Figs. 7A, 7B, and 7C.

With such a configuration, the brain wave measuring device 1 can act to be pressed downward against the head 99, and the electrode part 10 attached to the electrode fixing member 70 can be moderately pressed against the head 99.

### <<Second Embodiment>>

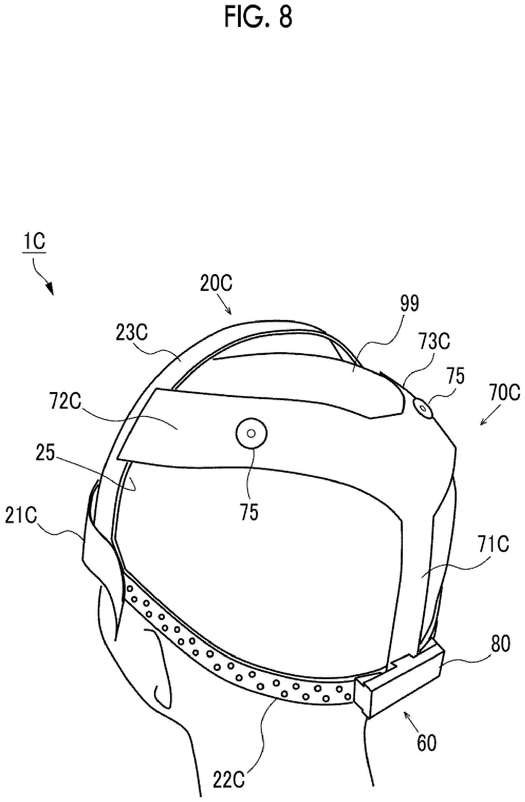

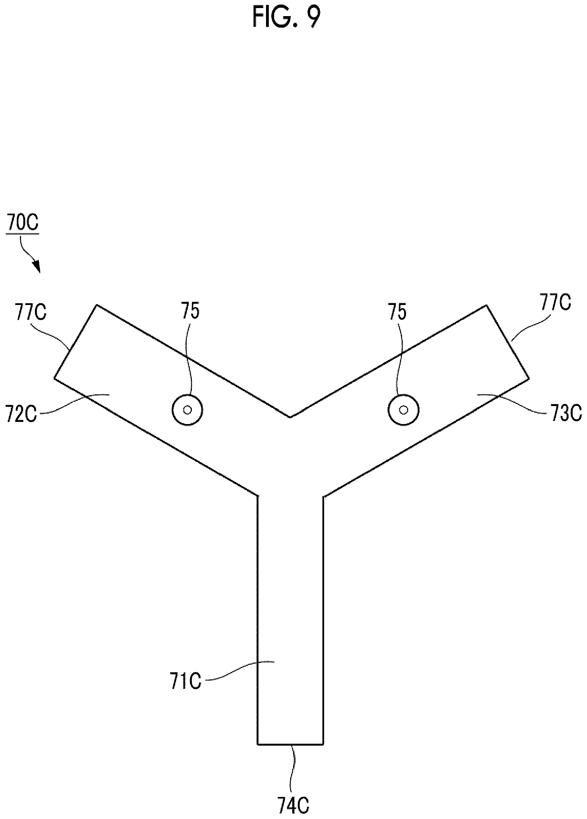

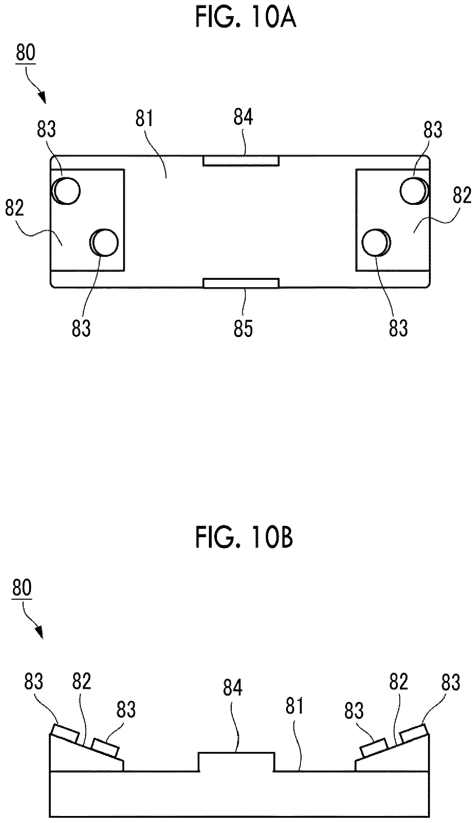

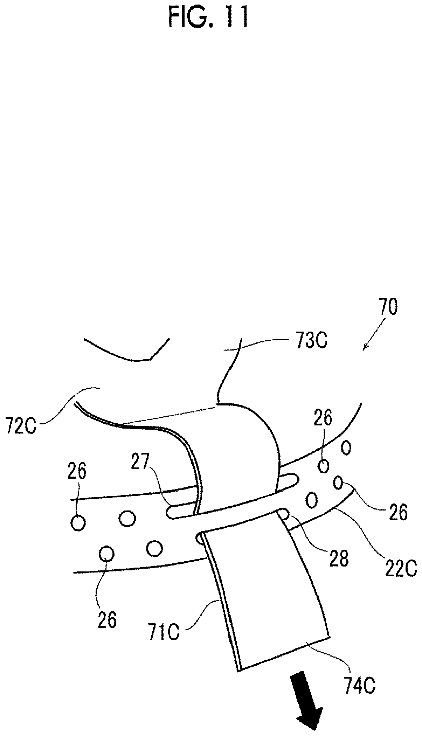

A brain wave measuring device 1C according to a second embodiment will be described with reference to Figs. 8 to 11. Fig. 8 is a diagram schematically showing the brain wave measuring device 1C in a state of being mounted on the head 99 of the subject. Fig. 9 is a plan view schematically showing the electrode fixing member 70C. Figs. 10A and 10B are diagrams schematically showing the fixing part 80, in which Fig. 10A is a plan view and Fig. 10B is a side view. Fig. 11 is a diagram schematically showing the fixed state of the fixing adjusting part 60, and particularly shows a state in which the first band part 71 of the electrode fixing member 70C is attached to the rear peripheral frame 22C.

### <Outline of Brain Wave Measuring Device 1C>

The brain wave measuring device 1C has a frame 20C, a band-shaped (or sheet-shaped) electrode fixing member 70C attached to the opening 25 on the occipital region side of the frame 20C, a fixing adjusting part 60 that is capable of adjusting a fixed state of the electrode fixing member 70C to the frame 20, and an electrode part (not shown) attached to the electrode fixing member 70C, as in the first embodiment. A difference between the present embodiment and the first embodiment is the attachment aspect of the electrode fixing member 70C to the frame 20C of the electrode fixing member 70C (that is, the fixing adjusting part 60), and the difference will be mainly described below, and the same configuration and function will be omitted from the description as appropriate.

### <Frame 20C>

The frame 20C is provided along the forehead, the temporal region, and the occipital region of the head 99 by combining a frame-shaped (band-shaped) hard member and an elastic member, and has a front peripheral band member 21C, a rear peripheral frame 22C, and a top frame 23C. Here, the front peripheral band member 21C is a band member having a hook and loop fastener mechanism and is provided to be adjustable in a fixed state on the forehead side. The top frame 23C is provided such that a head top portion is located on a forehead side, as compared with the first embodiment. In addition, details will be described later, but the rear peripheral frame 22C has an insertion part (first band insertion part 27 and second band insertion part 28) and the fitting opening 26, as a part of the configuration of the fixing adjusting part 60 (see Fig. 11).

### <Electrode fixing member 70C>

As shown in Fig. 9, the electrode fixing member 70C is a band member having a Y-shape in a plan view in a state of being removed from the frame 20C, and has a long band-shaped first band part 71C extending vertically in the diagram, and a second band part 72C and a third band part 73C that each are long band-shaped and extends out obliquely upward in two directions at one end of the first band part 71C on the upper side in the diagram. The material of the electrode fixing member 70C can be the same material as that of the electrode fixing member 70 according to the first embodiment.

The first band part 71C may have, for example, a width of 20 mm and a length of 150 mm.

The lower end part of the first band part 71C functions as a band adjusting part 74C that is inserted into and fixed to the first band insertion part 27 and the second band insertion part 28 of the frame 20 (rear peripheral frame 22C).

The second band part 72C extends out obliquely upward to the left in the drawing, and an extended out end part functions as a band attachment part 77C to be attached to the top frame 23C. The band attachment part 77C may be fixed to the top frame 23 by a screw, a string, or the like. In addition, the band attachment part 77C and the top frame 23C may be fixed to each other by providing a locking part in each of the band attachment part 77C and the top frame 23C. The second band part 72C may have, for example, a width of 30 mm and a length of 100 mm. The electrode attachment member 75 is provided at the substantially center of the second band part 72C. A position of the electrode attachment member 75 is, for example, an electrode position C3 in the 10-20 system.

The third band part 73C extends out obliquely upward to the right in the drawing, and an extended out end part functions as a band attachment part 77C to be attached to the top frame 23C. In addition, the band attachment part 77C and the top frame 23C may be fixed to each other by providing a locking part in each of the band attachment part 77C and the top frame 23C. The third band part 73C may have, for example, a width of 30 mm and a length of 100 mm. The electrode attachment member 75 is provided at the substantially center of the third band part 73. A position of the electrode attachment member 75 is an electrode position C4 in the 10-20 system.

### <Fixing Adjusting Part 60>

The fixing adjusting part 60 adjusts the expansion and contraction state of the electrode fixing member 70C to attach the electrode fixing member 70C to the frame 20C (rear peripheral frame 22C). Specifically, the fixing adjusting part 60 has the insertion part (first band insertion part 27 and second band insertion part 28) provided in the above-described frame 20C (rear peripheral frame 22C), the fitting opening 26, and the fixing part 80.

For example, as shown in Fig. 11, the rear peripheral frame 22C have a first band insertion part 27 and a second band insertion part 28 that each have an oblong opening at a position of the occipital region. The first band insertion part 27 is arranged on the upper side and the second band insertion part 28 is arranged on the lower side in a state of being arranged up and down. The opening sizes of the first band insertion part 27 and the second band insertion part 28 are set to a size into which the band adjusting part 74 can be inserted. The band adjusting part 74C of the electrode fixing member 70 is inserted into the first band insertion part 27 and the second band insertion part 28, and the band adjusting part 74 is fixed to the rear peripheral frame 22C by the fixing part 80.

The fitting opening 26 is a through-hole that is aligned and arranged in a zigzag shape in the horizontal direction in two rows in the upper and lower directions on the rear peripheral frame 22C. The fitting opening 26 has a shape in which a fitting protruding part 83 of a fixing part 80, which will be described below, is fitted exactly.

### <Fixing Part 80>

The fixing part 80 is made of, for example, a hard plastic, and fixes the electrode fixing member 70C (first band part 71C) inserted into the insertion parts (first band insertion part 27 and second band insertion part 28) to the frame 20C (rear peripheral frame 22C). Specifically, the fixing part 80 integrally has a base part 81, a support part 82, a fitting protruding part 83, and a band holding protruding part 84.

The base part 81 is a plate-shaped plate material and has support parts 82 in the vicinity of both end parts of one surface. The support part 82 is, for example, an inclined surface in which an outer side is higher and a center side is lower in the right-left direction, as shown in Fig. 10B. In a case where the electrode fixing member 70 is fixed by the fixing part 80, the inclined surface presses the band adjusting part 74 against the rear peripheral frame 22C. Two columnar fitting protruding parts 83 are provided to extend out from two positions of the inclined surface of the support part 82. The fitting protruding part 83 is fitted into the fitting opening 26 of the rear peripheral frame 22C described above in a case where the band adjusting part 74 is fixed by the fixing part 80.

In Fig. 10A, the band holding protruding parts 84 are provided at two portions in the vicinity of the upper and lower outer edges of the base part 81 to face each other in a rib shape. The distance between the two band holding protruding parts 84 is set to be substantially the same as (or slightly wider than) the width of the rear peripheral frame 22C. In a case where the electrode fixing member 70 is fixed by the fixing part 80, the band holding protruding parts 84 sandwiches and reliably fixes the band adjusting part 74 between the outer edge of the rear peripheral frame 22C.

### <Fixation of Electrode Fixing Member 70 by Fixing adjusting Part 60>

In a case where the electrode fixing member 70C is attached to the rear peripheral frame 22C, as shown in Fig. 10B, the electrode fixing member 70C is passed through the first band insertion part 27 from the outside to the inside and further passed through the second band insertion part 28 from the inside to the outside. Next, the band adjusting part 74 that protrudes outside of the second band insertion part 28 is pulled in a downward direction to adjust the expansion and contraction state of the electrode fixing member 70. After the adjustment of the expansion and contraction state of the electrode fixing member 70 is completed, the fixing part 80 is attached to the rear peripheral frame 22C to fix the band adjusting part 74. As a result, the inclined surface of the support part 82 of the fixing part 80 presses the band adjusting part 74 against the rear peripheral frame 22C, and the band adjusting part 74 is sandwiched by the band holding protruding parts 84 between the outer edge of the rear peripheral frame 22C. As a result, the band adjusting part 74 is reliably fixed to the rear peripheral frame 22C. In a case where the expansion and contraction state of the band adjusting part 74 is adjusted again, the fixing part 80 is removed, and the band adjusting part 74 is pulled to the outside or the inside.

The electrode fixing member 70C has a Y shape, but may have another shape. An appropriate shape can be selected according to a position at which the electrode attachment member 75 (that is, the electrode part 10) is attached. For example, the electrode fixing member 70C can be formed in an X shape, and the four positions of the electrode positions P3, P4, O1, and O2 in the 10-20 system may be set. At this time, the two upper arms of the X shape are fixed to the top frame 23, and the two lower legs are attached to the rear peripheral frame 22C at two locations to be adjustable in the expansion and contraction state. That is, the fixing adjusting part 60 is provided at two locations.

In addition, the electrode fixing member 70C may be integrally configured with the frame 20C. That is, the band adjusting part 74 of the first band part 71 may be configured to be separated and to be fixed by the fixing adjusting part 60 as described above as a configuration in which the second band part 72 and the third band part 73 of the electrode fixing member 70C are integrally provided with the top frame 23C.

The features of the present embodiment are summarized as follows.
(1) The brain wave measuring device 1 according to the present embodiment includes a frame 20 that is mounted on a head 99 of a subject, an electrode part 10 that comes into contact with the head 99, and an elastic electrode fixing member 70 which is attached to the frame 20 and to which the electrode part 10 is attached, in which the electrode fixing member 70 expands along a shape of the head 99 in a case where the frame 20 is mounted on the head 99 and the electrode part 10 is pushed against the head 99.
   Since the front peripheral band member 21 of the frame 20 expands and contracts, the frame 20 can be easily mounted on the head circumference. In addition, since the electrode fixing member 70 to which the electrode part 10 is attached is provided to cover the occipital region, all the electrode parts 10 can comes into contact with the head 99 with an appropriate force in a case where the brain wave measuring device 1 is mounted. That is, the time for appropriately abutting the electrode part 10 on the head 99 can be shortened.
(2) The electrode fixing member 70 has an elastic sheet material. As a result, the predetermined region of the head 99 can be covered integrally, and the electrode part 10 can be pressed against the head 99 as a whole.
(3) The electrode fixing member 70 has an elastic band member (horizontal band member 78 and vertical band member 79). As a result, the band member can be detached by a unit of the band member. In addition, since a gap can be formed, the air permeability is improved.
(4) A fixing adjusting part 60 that adjusts the expansion and contraction state of the electrode fixing member 70C to attach the electrode fixing member 70C to the frame 20C (rear peripheral frame 22C) is provided.
   As a result, the expansion and contraction state of the electrode fixing member 70C, that is, a state in which the electrode part 10 is pressed against the head 99 can be adjusted.
(5) The fixing adjusting part 60 has an insertion part (the first band insertion part 27 and the second band insertion part 28) that is provided in the frame 20C (22C) and into which a part of the electrode fixing member 70C (the first band part 71C) is inserted, and a fixing part 80 that fixes the electrode fixing member 70C (the first band part 71C) inserted into the insertion part (the first band insertion part 27 and the second band insertion part 28) to the frame 20C (the rear peripheral frame 22C).
   As a result, the expansion and contraction state of the electrode fixing member 70C, that is, the state in which the electrode part 10 is pressed against the head 99 can be adjusted with a simple configuration. In particular, by making the fixing part 80 easy to attach to and detach from the frame 20 (the frame 20c), the adjustment work can be simplified.
(6) The electrode fixing member 70 is attachable to and detachable from the frame 20, and an attachment position thereof is adjustable. As a result, the position of the electrode part 10 can be adjusted according to the size or the shape of the head 99.
(7) The electrode fixing member 70 and the frame 20 are integrally provided. As a result, the number of components can be reduced.
(8) A portion that expands and contracts in the electrode fixing member 70 is provided with a silicone rubber.
   As a result, it is possible to enhance the deterioration resistance during use and to ensure the flexibility.
(9) A mounting adjusting part 90 that adjusts a mounting state of the frame 20 in a head circumference direction.
   As a result, the mounting state of the brain wave measuring device 1 on the head 99 can be stabilized without making the subject feel uncomfortable.
(10) The brain wave measuring method according to the present embodiment includes performing a brain wave measurement by mounting the brain wave measuring device 1 described above on a head 99 of a subject.

This application claims priority on the basis of Japanese Patent Application No. 2021-203139 filed on December 15, 2021, the entire disclosure of which is incorporated into the present specification.

### REFERENCE SIGNS LIST

1, 1A, 1B, 1C brain wave measuring device
10 electrode part
20, 20C frame
21, 21C front peripheral band member
22, 22C rear peripheral frame
23, 23C top frame
25 opening
26 fitting opening
27 first band insertion part
28 second band insertion part
29 connecting part
30 electrode part main body
31 base
32 projecting part
33 conductive contact part
34 signal line part
49 female type snap button
50 cap-integrated type snap button
51 cap top plate
52 cap barrel part
53 male type snap button
60 fixing adjusting part
70, 70A, 70B, 70C electrode fixing member
71C first band part
72C second band part
73C third band part
74C band adjusting part
75 electrode attachment member
77C band attachment part
80 fixing part
81 base part
82 support part
83 fitting protruding part
84 band holding protruding part

## Claims

1. A brain wave measuring device comprising:
a frame that is mounted on a head of a subject;
an electrode part that comes into contact with the head; and
an elastic electrode fixing member that is attached to the frame and to which the electrode part is attached,
wherein the elastic electrode fixing member expands along a shape of the head in a case where the frame is mounted on the head and the electrode part is pushed against the head.

2. The brain wave measuring device according to Claim 1, wherein the elastic electrode fixing member has an elastic sheet material.

3. The brain wave measuring device according to Claim 1 or 2, wherein the elastic electrode fixing member has an elastic band member.

4. The brain wave measuring device according to Claim 2 or 3, further comprising:
a fixing adjusting part that adjusts an expansion and contraction state of the electrode fixing member and attaches the elastic electrode fixing member to the frame.

5. The brain wave measuring device according to Claim 4,
wherein the fixing adjusting part has
an insertion part that is provided in the frame and into which a part of the elastic electrode fixing member is inserted, and
a fixing part that fixes the elastic electrode fixing member inserted into the insertion part to the frame.

6. The brain wave measuring device according to any one of Claims 1 to 5,
wherein the elastic electrode fixing member is attachable to and detachable from the frame, and an attachment position is adjustable.

7. The brain wave measuring device according to any one of Claims 1 to 6,
wherein the elastic electrode fixing member and the frame are integrally provided.

8. The brain wave measuring device according to any one of Claims 1 to 7,
wherein a portion that expands and contracts in the elastic electrode fixing member is provided with a silicone rubber.

9. The brain wave measuring device according to any one of Claims 1 to 8, further comprising:
a mounting adjusting part that adjusts a mounting state of the frame in a head circumference direction.

10. A brain wave measuring method comprising:
performing a brain wave measurement by mounting the brain wave measuring device according to any one of Claims 1 to 9 on a head of a subject.
